# EUROPÄISCHE PATENTSCHRIFT

(11) **EP 2 004 081 B1**
(45) Veröffentlichungstag und Bekanntmachung des Hinweises auf die Patenterteilung: **18.05.2011**
(21) Anmeldenummer: 07727003.1
(22) Anmeldetag: 16.03.2007
(51) Int. Cl.: A61B 18/22

(54) **LASERAPPLIKATOR**
LASER APPLICATOR
APPLICATEUR DE LASER

(30) Priorität: 11.04.2006 DE 102006016957
(43) Veröffentlichungstag der Anmeldung: 24.12.2008
(73) Patentinhaber: Vimecon GmbH, 52134 Herzogenrath (DE)
(72) Erfinder: MARKUS, Kai, Ulf, 52477 Alsdorf (DE)
(74) Vertreter: von Kreisler Selting Werner
(86) Internationale Anmeldenummer: PCT/EP2007/052524
(87) Internationale Veröffentlichungsnummer: WO 2007/118745

(56) Entgegenhaltungen:
- EP-A1- 0 689 797
- WO-A-96/07451
- WO-A1-2006/019510
- DE-C1- 4 425 195
- US-B2- 6 676 656

## Beschreibung

Die Erfindung betrifft einen Laserapplikator mit einer Lichtleitstrecke, die in einer strangförmigen Umhüllung verläuft und in einem distalen Endabschnitt einen lateralen Auskoppelbereich aufweist, wobei die Umhüllung im Bereich des Endabschnitts zu einer Schleife geformt ist, deren Ebene quer zu dem Hauptteil der Umhüllung verläuft.

Vorhofflimmern ist die häufigste Herzrhythmusstörung in Europa und Nordamerika, die mehr als jeden 15. Menschen über 60 Jahre betrifft. Es entstehen in den Herzvorkammern (Vorhöfen) elektrische Erregungswellen, die sich chaotisch ausbreiten und die Pumpfunktion des Herzens behindern. Typische Beschwerden sind subjektiv empfundene Herzrhythmusstörungen, Herzrasen sowie Einschränkung der körperlichen Belastbarkeit, Schwindel sowie Ohnmachtsanfälle. Ohne Behandlung treten Schlaganfälle auf, die oft schwerwiegende Folgen bis hin zum Tod nach sich ziehen, da aufgrund der mangelnden Wandbewegung beim Vorhofftimmern Blutgerinsel entstehen können, die Embolien verursachen.

Kathetertechnische Verfahren zur Behandlung des Vorhofflimmerns bieten eine Chance auf einen dauerhaften Behandlungserfolg ohne Notwendigkeit der weiteren dauerhaften Medikamenteneinnahme. Hier wird der Ursprung der Rhythmusstörung im Herzen mittels eines dünnen flexiblen Katheters aufgesucht und verödet.

Bei den kathetertechnischen Verfahren werden bestimmte elektrisch aktive Bereiche des Vorhofgewebes aufgesucht und mittels Stromabgabe verödet. Ein Ausschalten oder Isolieren dieser Bereiche kann das Auftreten von Vorhofflimmern in ca. 60 - 80 % der Fälle verhindern. Hierzu werden mit multiplen punktuellen Stromabgaben in der linken Herzvorkammer kreisrunde Narben erzeugt, die das kranke Herzgewebe elektrisch vom Rest isolieren. Man nennt dieses Verfahren "Pulmonalvenenisolation". Ein herkömmlicher Katheter wird dazu zunächst in die rechte Herzvorkammer geführt. Um auf die linke Seite zu kommen, wird die Vorhofscheidewand durchstochen (Transseptale Punktion). Durch die Limitation der herkömmlichen Katheter auf punktuelle Läsionen müssen zur Erzeugung einer Isolationslinie unter 2-dimensionaler Röntgenkontrolle Punkte im 3-dimensionalen zu Raum Kreisen assembliert werden, ohne dabei Zwischenräume zu lassen oder gesundes Gewebe zu schädigen. Neuere Techniken zielen darauf, kreisrunde Läsionen um die Pulmonalvenen mit Ballonsystemen zu erreichen, die an den Pulmonalvenen expandiert werden und in diese hineinragen. Die Energieabgabe (z. B. Ultraschall, Laserenergie, Kälte) erfolgt im Inneren des Ballons und wird an die Außenseite geleitet. Das erfindungsgemäße System arbeitet ohne Ballon und kann daher sehr viel kleiner sein.

Ein Laserapplikator, von dem der Oberbegriff des Patentanspruchs 1 ausgeht, ist beschrieben in US 6,676,656 B2. Dieser Laserapplikator dient u. a. zur Behandlung von Arrhythmien. Er weist ein handgehaltenes Gehäuse auf, von dessen distalem Ende eine Umhüllung ausgeht, welche einen Lichtleiter enthält. Die Umhüllung kann um mehrere Pulmonarvenen herumgeschlungen werden, wobei aus dem Auskoppelbereich des Lichtleiters Strahlung auf die umschlossenen Pulmonarvenen fällt und diese verödet. Der Laserapplikator ist nur zur Verwendung am offenen Herzen geeignet, insbesondere in Verbindung mit dem Setzen von Bypässen. Hierdurch wird die Anwendbarkeit stark eingeschränkt. Ferner ergebene sich größere Abstände zwischen dem Lichtleiter und dem zu behandelnden Zielbereich, so dass eine relativ hohe Strahlungsleistung erforderlich ist.

In DE 198 03 460 C1 ist eine Applikationsvorrichtung für die Behandlung biologischer Gewebe mit Laserstrahlung beschrieben. Ein Lichtleiter, der von einer Mantelschicht umgeben ist, ist in einem distalen Endabschnitt mindestens teilweise von der Mantelschicht befreit, derart, dass die Laserstrahlung seitlich mit homogenem Verlauf aus dem Faserkern austritt. Der Lichtleiter enthält an den Austrittsstellen lichtstreuende Partikel. Dabei kann die Intensitätsverteilung der aus dem Strang austretenden Energie durch Veränderung der Partikeldichte beeinflusst werden.

DE 101 29 029 A1 beschreibt das Einbringen von Laserlicht in eine Lichtleiterstrecke, die Streukörper enthält zur Erzielung einer diffusen lateralen Abstrahlung.

In DE 44 07 498 C2 ist ein Lichtwellenleiter für Beleuchtungszwecke beschrieben, der das Licht lateral auskoppelt. Die Oberfläche des optischen Mantels ist zu diesem Zweck strukturiert.

Schließlich beschreibt EP 1 527 798 A2 einen Laserapplikator für die Fototherapie, der in einem distalen Endabschnitt ebenfalls Streupartikel in der Lichtleitstrecke enthält.

Der Erfindung liegt die Aufgabe zugrunde, einen Laserapplikator zu schaffen, mit dem ein Blutgefäß oder ein rohrförmiges Organ ringförmig verödet werden kann, um durch Isolation das Leiten elektrischer Signale zu unterbinden.

Der Laserapplikator nach der vorliegenden Erfindung zeichnet sich durch die Merkmale des Patentanspruchs 1 aus. Hiernach ist die Umhüllung der Lichtleiterstrecke Bestandteil eines langgestreckten flexiblen Katheters, der streckbar ist, um durch eine Schleuse oder über einen Führungsdraht an den Zielort vorgeschoben zu werden. Der Laserapplikator hat etwa die Form eines fliegenden Lassos. Er bildet an seinem Ende eine Schleife, die in einem Blutgefäß und/oder Hohlorgan derart platziert werden kann, dass sie in Kontakt mit der Umfangswand steht.

Durch laterale Auskopplung der Laserenergie aus der Lichtleitstrecke wird die Laserenergie gezielt in das benachbarte Gewebe eingeleitet. Der Laserapplikator kann wie ein üblicher Katheter intravaskulär in das Herz eingeführt werden, d. h. durch das Blutgefäßsystem des Patienten, so dass eine Öffnung des Herzens nicht erforderlich ist. Der Eingriff kann daher minimal-invasiv erfolgen.

Der erfindungsgemäße Laserapplikator eignet sich insbesondere zur Behandlung von Vorhofflimmern. Es kann Herzgewebe durch Umwandlung von Lichtenergie in thermische Energie veröden. Durch die aus der Lichtleitstrecke austretende Laserstrahlung wird das umgebende Gewebe auf Werte von über 60°C erhitzt, was zu einer Denaturierung von Proteinen und zum Ausbilden einer elektrisch inaktiven Narbe führt. Laserlicht dringt dabei in das Körpergewebe ein und wird an den Chromophoren (z. B. Blut- und Muskelfarbstoffe, Hämoglobin, Myoglobin) durch Absorption in Wärme umgewandelt. Dabei dringt das Licht in Abhängigkeit von der Chromophorenkonzentration und der Lichtwellenlänge einige Millimeter in das Gewebe ein, wobei es jedoch stark gestreut wird. Insbesondere zur Behandlung von Herzgewebe etwa bei Ablation zur Behandlung von Herzrhythmusstörungen, wird gefordert, dass sich die maximale Ausdehnung der Wärmeschädigung und damit der Narbe nicht direkt an der Applikation der Energie, der Innenseite des Herzens, befindet. Hier spielen die Interaktionen mit dem fließenden Blut und einer eventuellen Kühlung mittels austretender Flüssigkeit, typischerweise physiologische Kochsalzlösung, eine große Rolle.

Weiterhin ist es von Bedeutung bei vielen Herzrhythmusstörungen zusammenhängende Areale durch die Erzeugung von linienförmigen, kreisförmigen oder sonstig gestalteten Narben elektrisch von anderen zu isolieren. Heutzutage wird dies meist durch die Aneinanderreihung von punktuellen Läsionen (meist mit Stromabgabe) erreicht.

Das erfindungsgemäße System dient dazu, Laserlicht so aus einem Katheter auszuleiten ("auszukoppeln"), dass linienförmige, kreisförmige oder sonstig gestaltete Areale mit dieser Energie behandelt werden können, ohne das Kathetersystem oder einzelne Komponenten in dem System während der Energieapplikation bewegen oder in der Lage verändern zu müssen.

Die Schleife beschreibt einen Umfangsbereich von mehr als 180°. Vorzugsweise erstreckt sie sich über 360°, so dass an einem Gefäß oder Hohlorgan eine geschlossen ringförmige Narbe erzielt wird. Eine geschlossene Ringform ist aber nicht in allen Fällen notwendig.

Der Laserlichtapplikator ist flexibel, hat jedoch eine Vorformung bzw. ein Formgedächtnis. Zum Einbringen in den Körper wird er zwangsweise gestreckt und beispielsweise durch eine Schleuse oder über einen Führungsdraht an den Zielort vorgeschoben. Danach wird die Führungsvorrichtung entfernt, wobei der Katheter eine vorgegebene Grundform annimmt.

Gemäß einer bevorzugten Ausgestaltung der Erfindung ist vorgesehen, dass der Auskoppelbereich im Querschnitt des Stranges von der Schleife aus überwiegend oder ausschließlich nach außen gerichtet ist. Dabei wird bei Anlage des Katheters am Gewebe kurz unterhalb der Gewebeoberfläche die höchste Energiedichte erreicht. Dies ist wichtig, um Denaturierungsprozesse und Karbonisierung der Blutbestandteile und der Gewebeoberfläche zu minimieren, die abhängig von der Energiedichte sind.

Außer der Formung eines Kreises zur Bildung kreisrunder Koagulationsnegrosen kann die Schleife auch so ausgebildet sein, dass Linien, Bögen oder andere Bauformen entstehen. Dies kann zweckmäßig sein, um z. B. Pulmonalvenen bei Vorhofflimmern elektrisch zu isolieren sowie linksatriale Substratmodifikationen durchzuführen.

Die laterale Auskopplung der Laserstrahlung aus der Lichtleitstrecke kann auf verschiedene Arten erfolgen. Hierzu bietet der Stand der Technik zahlreiche Möglichkeiten. Eine dieser Möglichkeiten sieht vor, dass der Brechungsindex des den Lichtleiterkern umgebenden Mantels an der Auskoppelstelle kleiner gemacht wird als in dem übrigen Bereich, so dass im Auskoppelbereich keine Totalreflexion mehr auftritt und das Licht seitlich austritt. Eine andere Möglichkeit besteht darin, den Brechungsindex des umgebenden Mantels an der Austrittsstelle höher zu machen als denjenigen des Faserkems im transmissiven Teil der Lichtleitphase.

Die gerichtete Auskopplung der Energie aus dem Strang kann ebenfalls auf unterschiedliche Arten erfolgen, beispielsweise indem ein längslaufender Streifen des Umfangs des Fasermantels einen anderen Brechungsindex hat als der übrige Mantelumfang. Erreicht werden kann dies durch Extrusion unterschiedlicher Materialien. Alternativ hierzu kann ein Teil des Umfanges des Mantelmaterials abgetragen oder mit der darunter liegenden Kernfaserschicht verschmolzen werden, was zu einer Veränderung des Brechungsindex an dieser Stelle führt. Ein weiteres Mantelmaterial, z. B. mit höherem Brechungsindex, kann auf die nunmehr teilweise ummantelte Kernfaser aufgetragen werden.

Eine weitere Möglichkeit, die eine Lichtauskopplung durch Änderung der Brechungsindizes von Faserkern und Mantel bewirkt, ist die Anbringung eines weiteren Materials mit höherem Brechungsindex an dem nicht ummantelten Faserkernmaterial. Der Rest des Kreissegments wird von einem Material mit niedrigerem Brechungsindex umgeben, das Licht an der Grenzfläche reflektiert. Insbesondere kann diese Anordnung erfolgen, indem eine Kernfaser in einen Katheterschlauch eingebracht und an der Schlauchwand mit höherem Brechungsindex angebracht wird. Das übrigen Lumen wird mit einem anderem Material mit niedrigerem Brechungsindex aufgefüllt, z. B. mit Wasser, das auch zu Spülzwecken verwendet werden kann.

Schließlich besteht die Möglichkeit, den Austrittswinkel des Lichtes aus der Faser zu begrenzen, indem Materialien auf die Oberfläche aufgebracht werden.

Um eine über die Länge der Auskoppelstrecke gleichmäßige Energieverteilung zu erreichen, kann der Winkel des Mantel-Kreissegments, der die Auskopplung bewirkt, über die Länge der Auskoppelstrecke verändert werden. Ferner kann der Winkel des Kreissegments durch eine Reflektionsschicht bestimmt werden, die auf der Kernfaser oder dem Fasermantel angebracht wird.

Eine alternative Möglichkeit der Beeinflussung der austretende Strahlungsintensität besteht darin, den Refraktionsindex-Koeffizienten zwischen Kern und Mantel über die Länge des Auskopplungsbereiches zu verändern. Weiterhin kann dies erreicht werden, durch das Einbringen von zusätzlichen Materialien, z. B. Siliciumdioxid-Nanopartikel, in einen Kunststoff, so dass der Brechungsindex sich in axialer Richtung verändert. Schließlich besteht auch die Möglichkeit, Silikon oder eine andere Masse in viskoser Phase in den Katheterschlauch einzubringen.

Eine weitere Möglichkeit der Beeinflussung der austretenden Strahlungsenergie besteht in der Einbringung von Stufenauskopplern über die Stranglänge des Laserlichtapplikators. Stufenauskoppler bewirken eine stufenweise Teilauskopplung der Laserenergie aus einer Lichtleitphase durch Einbringen von Einkerbungen in die Kernfaseroberfläche. Am Faserende befindet sich ein Reflektor, der den restlichen Anteil des Laserlichts in die Faser reflektiert.

Schließlich kann eine Regelung der austretenden Strahlungsenergie mittels Reflexion oder Absorption im Fasermaterial erfolgen.

Der Laserapplikator sollte eine möglichst große Flexibilität haben. Andererseits ist der Biegeradius von Lichtleitfasern begrenzt. Zur Lösung dieses Problems kann die Lichtleitstrecke mehrere parallele Lichtleitfasern enthalten. Da die Energie auf mehrere Fasern "verteilt" werden muss, um das maximale Leistungsflächenprodukt der einzelnen Lichtleitfaser nicht zu überschreiten, kommen hier mehrere Fasern mit einem Kerndurchmesser von z. B. 10 - 50 µm zum Einsatz, die ihre Energie durch seitliche Auskopplung in den Laserapplikator und dann in das umliegende Körpergewebe abgeben. Diese Fasern können nebeneinander liegen und jeweils alle über die gesamte Länge des Laserapplikators Energie abgeben. Alternativ können die Fasern so angeordnet sein, dass jeweils eine Faser einen Teil der Länge des Applikators mit Laserenergie versorgt und dann endet. Eine weitere Faser, die im Applikator innen liegt, wird nach außen an den Applikator geführt und gibt auf einem weiteren Teilstück der Gesamtlänge der Laserlichtapplikation Laserlicht ab. Beide Möglichkeiten können in Kombination verwendet werden, um axial und radial eine gleichmäßige Auskopplung des Laserlichts aus dem Laserlichtapplikator zu gewährleisten.

Da die Einkoppelverluste bei Verwendung eines Diodenlasers hoch sind, eignet sich diese Anordnung speziell für die Verwendung eines Faserlasersystems.

Bei der Anwendung von Laserlicht, das aus einer Lichtleitfaser ausgekoppelt wird, nimmt die maximale Energiedichte des Laserlichtes in der durch den Radius der Lichtleitfaser bestimmten Ebene mit zunehmender Entfernung von dem Auskoppelort ab und ist normalerweise bei am Ort der Auskopplung am höchsten. Das kann jedoch dazu führen, dass Strukturen, die nahe an dem Laserapplikator liegen, hohe thermische Energien aufnehmen müssen, damit in der Tiefe der gewünschte Effekt erzielt wird. Insbesondere kann es an der Gewebsoberfläche zur Koagulation und Karbonisierung kommen. Da karbonisierte Flächen Laserlicht vollständig absorbieren und damit den unerwünschten Effekt der übermäßigen Erwärmung an der Oberfläche verstärken, sollte dieser Effekt verhindert werden.

Gemäß einer bevorzugten Ausgestaltung der Erfindung wird die Lichtstrahlung auf einen Brennpunkt fokussiert, der entfernt von dem Applikator und der Katheteroberfläche in der Tiefe des Gewebes liegt. Durch Streuung im Gewebe ist eine genau definierte Fokussierung nicht möglich. Dennoch kann durch Fokussierung die Vorzugsrichtung der Strahlausbreitung im Gewebe definiert werden. Es ergibt sich ein Wirkungs- und Absorptionsmaximum, das zu einer Gewebeerwärmung typischerweise in einigen Millimetern Entfernung vom Applikator führt.

Im Folgenden werden unter Bezugnahme auf die Zeichnungen Ausführungsbeispiele der Erfindung näher erläutert.

Es zeigen:
- Fig. 1: eine schematische Darstellung des Laserapplikators in einem Blutgefäß,
- Fig. 2: in vergrößertem Maßstab einen Schnitt entlang der Linie II-II von Figur 1,
- Fig. 3 - 7: Querschnitte durch weitere Ausführungsformen des Laserapplikators und
- Fig.8: eine Ansicht eines keilförmigen Auskoppelbereichs zur Erzielung einer in Längsrichtung gleichmäßigen Verteilung der ausgekoppelten Energie.

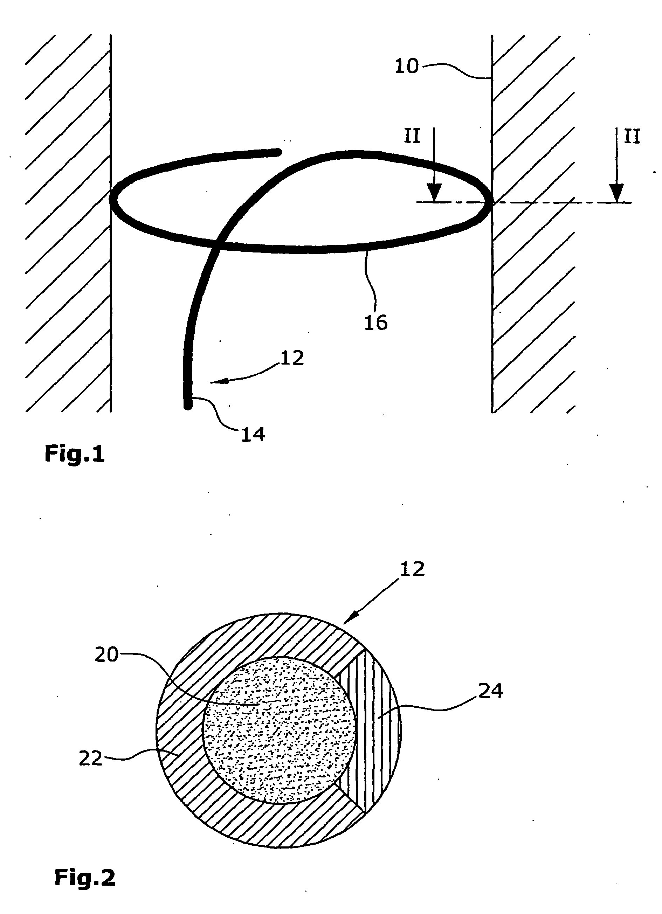

Figur 1 zeigt ein rohrförmiges Blutgefäß 10, dessen Wand mit dem Laserapplikator thermisch behandelt werden soll. Der Laserapplikator weist einen Katheter 12 in Form eines langgestreckten Stranges auf. Der Katheter kann eines oder mehrere Lumen enthalten, jedoch kann er auch hohlraumfrei sein. Der Katheter 12 ist in der in Figur 1 schematisch dargestellten Weise vorgeformt. Er weist einen Hauptteil 14 auf, der im wesentlichen gerade ist, und eine Schleife 16, die einen an einer Stelle offenen Kreis bildet. Während der Hauptteil 14 in dem Blutgefäß longitudinal verläuft, legt sich die Schleife 16 in dem Blutgefäß quer, so dass sie ringförmig an der Wand anliegt. Die Schleifenebene liegt quer, insbesondere rechtwinklig, zur Längsrichtung des Hauptteils 14. Die Schleife hat somit eine solche Größe, dass sie sich von innen mit leichtem Druck gegen die Wand eines Blutgefäßes legt. Der Außendurchmesser der Schleife beträgt etwa 4-6mm.
Figur 2 zeigt einen Querschnitt durch den Strang des Katheters 12. Der Strang enthält einen Kern, der hier aus einer einzigen Lichtleitfaser 20 besteht, und einen Mantel 22 von höherem Brechungsindex als der Kern. Bei dem vorliegenden Ausführungsbeispiel erstreckt der Mantel sich über einen Umfangsbereich des Kernes von über 270°. Der verbleibende Teil des Umfangs wird von einem Auskoppelbereich 24 gebildet, der hier aus einem Material mit kleinerem Brechungsindex als der Mantel 22 besteht. Aus dem Segment des Auskoppelbereichs 24 tritt die Strahlung lateral aus dem Strang aus. Die Strahlung tritt also nur nach einer Seite des Strangumfanges hin aus.

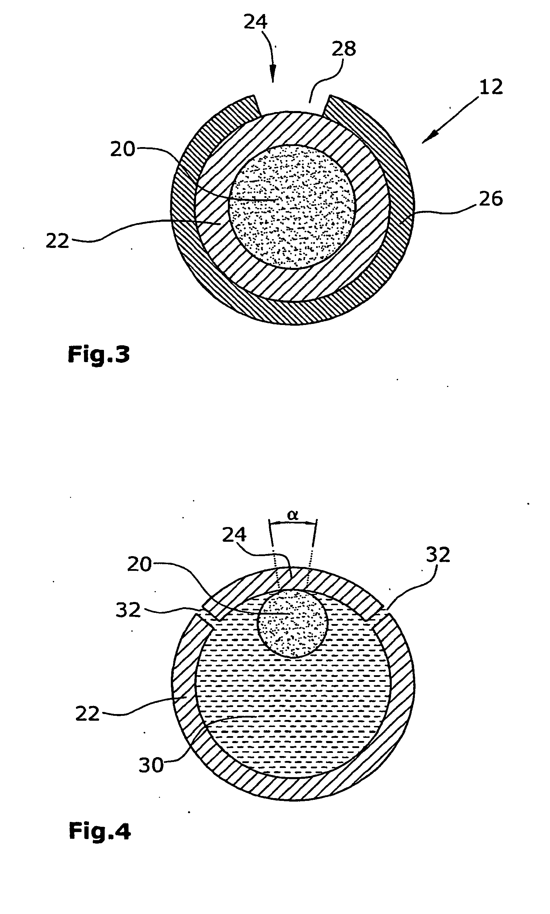

Figur 3 zeigt ein Ausführungsbeispiel, bei dem der Mantel 22 aus lichtdurchlässigem Material von einer Reflexionsschicht 26 umgeben ist. Diese lässt ein Fenster 28, welches den Auskoppelbereich 24 bildet, frei, so dass auch hier das Licht nur an einer Stelle des Umfangs austritt.
Figur 4 zeigt ein Ausführungsbeispiel, bei dem die Lichtleitfaser mit einem Teil ihres Umfangs unmittelbar an dem Mantel 22 anliegt, so dass an dieser Stelle eine Grenzschicht zwischen beiden entsteht. Die Lichtleitfaser 20 ist exentrisch in dem Mantel 22 angeordnet und der freie Raum bildet ein Lumen 30, welches mit einem Material mit niedrigem Brechungsindex gefüllt ist, beispielsweise mit Wasser. Der Mantel 22 enthält Spülkanäle 32, durch die Flüssigkeit aus dem Lumen 30 nach außen austreten kann.
   Durch die Berührung zwischen der Lichtleitfaser 20 und dem Mantel 22 entsteht ein lateraler Auskoppelbereich 24 mit einem Öffnungswinkel a .

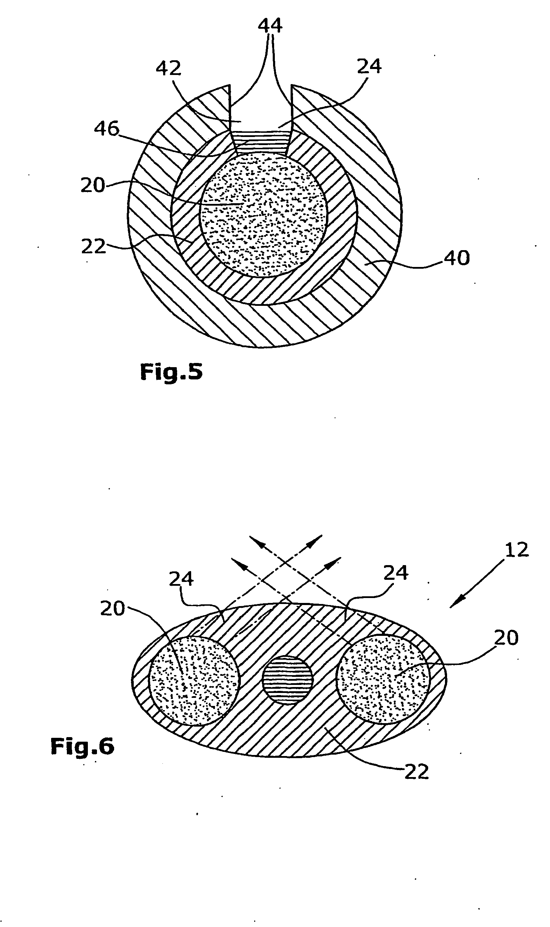

Figur 5 zeigt ein Ausführungsbeispiel, bei dem der Mantel 22 der Lichtleitfaser 20 mit einem äußeren Schutzmantel 40 umgeben ist. An der Stelle, an der sich der Auskoppelbereich 24 befindet, ist der Schutzmantel 40 mit einem Schlitz 42 versehen, dessen Wand mit einer Reflektorschicht 44 beschichtet ist. Im Auskoppelbereich 24 enthält der Mantel 22 einen Diffusor 46 zum lateralen Auskoppeln der Laserenergie.
In Figur 6 ist der Querschnitt eines Katheters 12 dargestellt, der in einem Mantel 22 mehrere Lichtleitfasern 20 eingebettet enthält. Die Lichtleitfasern 20, deren Licht in den Auskoppelbereichen 24 aus dem Mantel 22 austritt, bündeln das ausgekoppelte Laserlicht in einer Entfernung vom Katheter 12, wobei an der Kreuzungsstelle der Strahlen die maximale Energiedichte erreicht wird.

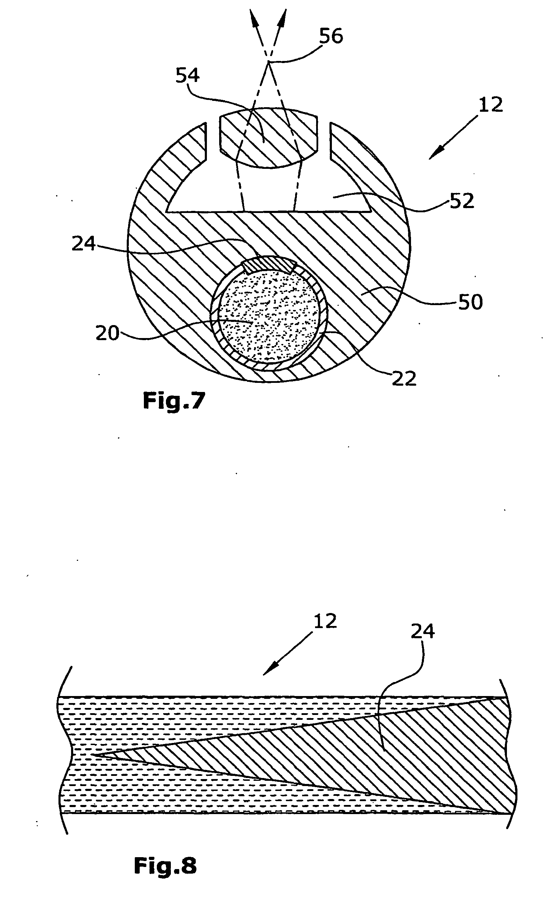

Figur 7 zeigt einen Katheter 12 mit Lichtleitfaser 20 und Mantel 22, wobei der Auskoppelbereich 24 gemäß Figur 2 ausgebildet ist. Die Lichtleitstrecke ist mit einem Außenmantel 50 umgeben und in diesem exentrisch angeordnet. Der Außenmantel 50 enthält ein Lumen 52, das beispielsweise eine Spülflüssigkeit enthält. Diese hat einen geringeren Brechungsindex als der Außenmantel 50, so dass der Außenmantel eine Linse 54 bildet, durch die die aus dem Auskoppelbereich 24 ausgekoppelte Strahlung auf einen Fokus 56 fokussiert wird.
In Figur 8 ist ein Katheter dargestellt, bei dem der Auskoppelbereich 24 sich in Längsrichtung des Katheters 12 verbreitert, so dass der Auskoppelbereich insgesamt etwa keilförmig ausgebildet ist. Dadurch wird eine gleichmäßige Abstrahlcharakteristik in axialer Richtung und eine diskontinuierliche Abstrahlung in radialer Richtung erreicht.

## Patentansprüche

1. Laserapplikator mit einer Lichtleitstrecke (20), die in einer strangförmigen Umhüllung verläuft und in einem distalen Endabschnitt einen lateralen Auskoppelbereich (24) aufweist, wobei die Umhüllung im Bereich des Endabschnitts zu einer Schleife (16) geformt ist, deren Ebene quer zu dem Hauptteil (14) der Umhüllung verläuft,
**dadurch gekennzeichnet,**
**dass** der Laserapplikator einen langgestreckten flexiblen Katheter (12) aufweist, dessen Bestandteil die Umhüllung ist, und dass der Laserapplikator streckbar ist, um durch eine Schleuse oder über einen Führungsdraht an den Zielort vorschiebbar zu sein.

2. Laserapplikator nach Anspruch 1, **dadurch gekennzeichnet, dass** der Auskoppelbereich (24) im Querschnitt des Stranges von der Schleife (16) aus überwiegend oder ausschließlich nach außen gerichtet ist.

3. Laserapplikator nach Anspruch 1 oder 2, **dadurch gekennzeichnet, dass** die Schleife (16) einen Bereich von mehr als 180° beschreibt.

4. Laserapplikator nach einem der Ansprüche 1 - 3, **dadurch gekennzeichnet, dass** der Auskoppelbereich (24) sich über weniger als 90° des Umfangs der Lichtleitstrecke erstreckt.

5. Laserapplikator nach einem der Ansprüche 1 - 4, **dadurch gekennzeichnet, dass** der Strang einen rohrförmigen Mantel (22) mit einem darin verlaufenden Lichtleiter (20) aufweist.

6. Laserapplikator nach Anspruch 5, **dadurch gekennzeichnet, dass** der Mantel (22) ein Lumen (30) für eine mit dem Lichtleiter (20) in Kontakt bringbare lichtbrechende Flüssigkeit umschließt.

7. Laserapplikator nach Anspruch 6, **dadurch gekennzeichnet, dass** das Lumen (30) seitliche Austrittsöffnungen (32) aufweist.

8. Laserapplikator nach einem der Ansprüche 1 - 7, **dadurch gekennzeichnet, dass** der Auskoppelbereich (24) mit einer konzentrierenden Linse (54) versehen ist.

9. Laserapplikator nach einem der vorhergehenden Ansprüche, **dadurch gekennzeichnet, dass** der Auskoppelbereich (24) eine in distaler Richtung zunehmende Breite aufweist.

10. Laserapplikator nach einem der Ansprüche 1 - 9, **dadurch gekennzeichnet, dass** die Lichtleitstrecke mehrere Lichtleiter (20) enthält.

11. Laserapplikator nach Anspruch 10, **dadurch gekennzeichnet, dass** die Auskoppelbereiche (24) mindestens zweiter Lichtleiter (20) einander kreuzende Austrittsrichtungen aufweisen.

## Claims

1. Laser applicator comprising a photoconducting path (20) extending in a strand-shaped jacket and comprising a lateral output coupling portion (24) in a distal end section, the jacket being formed as a loop (16) in the region of the end section, the plane of the loop extending transversely to the main part (14) of the jacket,
**characterized in that**
the laser applicator comprises an elongate flexible catheter (12) having the jacket as one of its components, and that the laser applicator can be straightened so as to be advanced to the target site through a tube or over a guide wire.

2. The laser applicator of claim 1, **characterized in that** the decoupling portion (24) is mainly or exclusively directed outward from the loop (16), seen in cross section of the strand.

3. The laser applicator of claim 1 or 2, **characterized in that** the loop (16) describes a portion of more than 180°.

4. The laser applicator of one of claims 1-3, **characterized in that** the decoupling portion (24) extends over less than 90° of the circumference of the optical fiber.

5. The laser applicator of one of claims 1-4, **characterized in that** the strand comprises a tubular sheath (22) with an optical fiber (20) extending therein.

6. The laser applicator of claim 5, **characterized in that** the sheath (22) encloses a lumen (30) for a light-refracting liquid in contact with the optical fiber (20).

7. The laser applicator of claim 6, **characterized in that** the lumen (30) has lateral exit openings (32).

8. The laser applicator of one of claims 1-7, **characterized in that** decoupling portion (24) is provided with a focusing lens (54).

9. The laser applicator of one of the preceding claims, **characterized in that** the decoupling portion (24) has a width increasing in the distal direction.

10. The laser applicator of one of claims 1-9, **characterized in that** the optical fiber comprises a plurality of optical fibers (20).

11. The laser application of claim 10, **characterized in that** the decoupling portion (24) of at least two optical fibers (20) have exit directions crossing each other.

## Revendications

1. Applicateur de laser comprenant une liaison optique (20) qui s'étend dans une enveloppe en forme de boyau et comprend une partie de découplage latérale (24) dans une section d'extrémité distale, ladite enveloppe étant formée en une boucle (16) dans la région de ladite section d'extrémité, le plan dudit boucle s'étendant transversalement à la partie principale (14) de ladite enveloppe,
**caractérisé en ce que**
ledit applicateur de laser comprend un cathéter (12) élongé flexible, dont l'enveloppe forme une partie, et que ledit applicateur de laser est apte à être redressé afin d'être avancé jusqu'au lieu de destination à travers une gaine ou sur un fil de guidage.

2. Applicateur de laser selon la revendication 1, **caractérisé en ce que** ladite partie de découplage (24) est orientée pour la plupart ou exclusivement vers l'extérieur à partir de la boucle (16), vu en coupe transversale du boyau.

3. Applicateur de laser selon les revendications 1 ou 2, **caractérisé en ce que** ladite boucle (16) décrit une plage supérieure à 180°.

4. Applicateur de laser selon l'une quelconque des revendications 1-3, **caractérisé en ce que** ladite partie de découplage (24) s'étend sur moins que 90° de la périphérie de la liaison optique.

5. Applicateur de laser selon l'une quelconque des revendications 1-4, **caractérisé en ce que** ledit boyau comprend une enveloppe tubulaire (22) avec une fibre optique (20) s'étendant dans celle-ci.

6. Applicateur de laser selon la revendication 5, **caractérisé en ce que** ladite enveloppe (22) comprend une lumière (30) pour un liquide réfractif apte à être mise en contact avec ladite fibre optique (20).

7. Applicateur de laser selon la revendication 6, **caractérisé en ce que** la lumière (30) comprend des ouvertures de sortie (32) latérales.

8. Applicateur de laser selon l'une quelconque des revendications 1-7, **caractérisé en ce que** ladite partie de découplage (24) est munie d'une lentille concentrante (54).

9. Applicateur de laser selon l'une quelconque des revendications précédentes, **caractérisé en ce que** la largeur de ladite partie de découplage (24) croît dans la direction distale.

10. Applicateur de laser selon l'une quelconque des revendications 1-9, **caractérisé en ce que** ladite liaison optique comprend plusieurs fibres optiques (20).

11. Applicateur de laser selon la revendication 10, **caractérisé en ce que** les parties de découplage (24) d'au moins deux fibres optiques (20) présentent des directions de sortie convergentes.
